# EUROPEAN PATENT APPLICATION

(11) **EP 4 837 532 A1**
(43) Date of publication of application: **11.11.2026**
(21) Application number: 26169477.2
(22) Date of filing: 31.03.2026
(51) Int. Cl.: A61B 18/14

(54) **PLASMA RESECTOSCOPE**

(30) Priority: 08.05.2025 CN 202510587710
(71) Applicant: Hangzhou Hawk Optical Electronic Instruments Co., Ltd., Hangzhou, Zhejiang 311201 (CN)
(72) Inventor: WANG, Yanliang, Xiaoshan Hangzhou, 311201 (CN); ZHANG, Zhong, Xiaoshan Hangzhou, 311201 (CN); WANG, Lei, Xiaoshan Hangzhou, 311201 (CN); SHAO, Hualiang, Xiaoshan Hangzhou, 311201 (CN); LIN, Hai, Xiaoshan Hangzhou, 311201 (CN); HUA, Xueke, Xiaoshan Hangzhou, 311201 (CN)
(74) Representative: Metida

(57) **Abstract**

A plasma resectoscope including a sheath assembly, an operating unit, an electric resection loop assembly and an electronic endoscope, wherein the sheath assembly is disposed at a front end of the operating unit, the sheath assembly includes an outer sheath and an inner sheath, the outer sheath is sleeved on an outer side of the inner sheath, the electronic endoscope includes a lens tube and an operating handle, the lens tube is disposed within the inner sheath, the electric resection loop assembly includes a cutting tip, a connecting rod and an electrode rod, a slider is connected to a tail end of a rear side of the electrode rod, the slider is slidably installed on the operating unit, a finger hook is connected to the slider, and a distance sensor sensing a position of the slider is disposed on the operating unit. The present disclosure utilizes an electronic endoscope combined with a plasma electric resection loop. An electronic imaging system is mature, cost-effective, and simple in process. The electronic endoscope offers a large field of view angle, providing a wide visual range. Furthermore, the image sensor is small in size, allowing for a reduction in an overall outer diameter.

## Description

### BACKGROUND

### 1. Technical Field

The present disclosure relates to the technical field of medical devices, particularly to the technical field of plasma resectoscopes.

### 2. Description of Related Art

A plasma resectoscope utilizes energy generated by bipolar radiofrequency to convert an electrolyte solution between a radiofrequency cutting tip and a tissue into a thin plasma layer. These high-speed moving ions possess sufficient energy to break organic molecular bonds of a target tissue, causing macromolecular substances to disintegrate into small molecular substances and producing a vaporization effect, thereby realizing tissue resection. Moreover, during a sectioning process, arterioles, venules and capillaries in a deep layer can be rapidly closed, providing effective hemostasis.

In the prior art, resectoscopes typically employ optical endoscopes for imaging. However, the optical endoscopes are associated with high costs, complex manufacturing processes and limited field of view. Moreover, a diameter of a lens tube is inversely proportional to the image quality, with various technical parameters having reached a bottleneck. For instance, Chinese utility model patent with a publication number of CN221154288U discloses a resectoscope employing an optical endoscope for observation. Similarly, Chinese utility model patent with a publication number of CN215914871U discloses an electric rotating plasma resectoscope employing an optical endoscope for observation.

An electronic imaging system is mature, cost-effective, and simple in process. However, an electronic endoscope has not yet been applied toa resectoscope. The optical endoscope achieves image acquisition and display by means of a connection with an external camera, whereas a camera of the electronic endoscope is positioned at a front end of an endoscope body, applying the electronic endoscopes to the resectoscope presents the following challenges.
1. Optical-frequency interference emitted by an electric resection loop has a significant impact on a close-range camera, rendering the captured image more prone to artifacts such as streaks or noise, which leads to the unclear image.
2. Heat generated during operation of the electric resection loop has a significant impact on the camera, potentially damaging the camera.
3. The optical resectoscope is characterized by the rotation of the optical endoscope while the camera engaged to a rear side remains stationary, thereby ensuring image orientation. However, the camera rotates along with the electronic endoscope because of being installed in front of the electronic endoscope, causing the captured image to rotate accordingly. Rotating the image may lead to a deviation in the surgeon's perception of an operating direction.

### SUMMARY

An objective of the present disclosure is to solve the problem in the prior art and provide a plasma resectoscope that can solve the above problem.

In order to achieve the above objective, the present disclosure discloses a plasma resectoscope including a sheath assembly, an operating unit, an electric resection loop assembly and an electronic endoscope, wherein the sheath assembly is disposed at a front end of the operating unit, the sheath assembly includes an outer sheath and an inner sheath, the outer sheath is sleeved on an outer side of the inner sheath, the electronic endoscope includes a lens tube and an operating handle, the lens tube is disposed within the inner sheath; the electric resection loop assembly includes a cutting tip, a connecting rod and an electrode rod, the cutting tip is disposed on a front side of the lens tube, two sides of the cutting tip are connected to the connecting rod; the connecting rod is connected to the electrode rod, the electrode rod is disposed on two sides of the lens tube, a slider is connected to a tail end of a rear side of the electrode rod, the slider is slidably installed on the operating unit, a finger hook is connected to the slider, and a distance sensor sensing a position of the slider is disposed on the operating unit.

The distance sensor functions to ensure that an electric resection loop is activated only at a specific distance from a tip end of the electronic endoscope. When the slider of the electric resection loop is within a certain range of the sensor, the electric resection loop does not operate even if a switch of the electric resection loop is turned on, which solves the problem of thermal damage to the tip end of the electronic endoscope due to high temperature of the electric resection loop during misoperation when the electric resection loop is in excessive proximity to the tip end of the electronic endoscope.

Preferably, a conduit is disposed on two sides of the lens tube, the electrode rod is slidably sleeved in the conduit, a lens barrel is provided at a distal end of the lens tube, an image sensor is disposed in the lens barrel, a protective glass is disposed on an outer surface of the lens barrel, and a cut-off film is disposed on one side of the protective glass close to the image sensor.

The protective glass functions to reduce physical damage to the image sensor, thereby prolonging the service life of the image sensor.

A coating cutting off specific wavelengths is sprayed on the protective glass to eliminate or reduce the interference of specific light waves on a module.

Preferably, a distal tip is disposed within the lens barrel, the image sensor is installed in the distal tip, a diameter of the distal end of the image sensor is greater than a diameter of a proximal end of the image sensor, a diameter of the lens barrel is greater than a diameter of the lens tube, and the lens barrel and the lens tube are in smooth transition by means of an inclined surface.

The lens tube with a matching structure is fabricated by utilizing a characteristic of the image sensor having a large tip end and a thin wire at a rear end, allowing for optimized allocation of the inflow/outflow channel, and further effectively increasing the total inflow/outflow area.

Preferably, an installing slot for the protective glass is provided in a surface of the distal tip, a limiting groove for installing a lens barrel is provided in the middle of the installing slot, a first bonding surface and a second bonding surface are disposed in the installing slot, a left side and a right side of the installing slot are of an arc-shaped structure, the first bonding surface is bonded to a periphery of the bottom surface of the protective glass, the second bonding surface is bonded to a side surface of the protective glass, and a light guide bundle for illumination is disposed above the distal tip.

The light guide bundle is positioned above the image sensor, and a lower end of the image sensor is a flat end.

The first bonding surface and the second bonding surface function to improve the bonding firmness of the protective glass and the distal tip.

Preferably, the lens barrel is bent downwards and forms a viewing angle θ with a central axis of the inner sheath, and an installing direction of the image sensor is consistent with a bending direction of the lens barrel.

Preferably, the viewing angle θ ranges from 0 to 30°.

Preferably, a gap between the inner sheath and the outer sheath is an outflow channel, and a gap between the inner sheath and lens tube is an inflow channel.

Preferably, the distance sensor includes a photoelectric sensor, a spring, a fixed rod and a telescopic rod, wherein the fixed rod is fixed on the operating unit, an installing groove is disposed within the fixed rod, the photoelectric sensor is disposed on one end of the installing groove away from the slider, the telescopic rod is slidably installed on one end of the installing groove close to the slider, a tail end of the telescopic rod protrudes beyond the fixed rod, a limiting portion is disposed on the telescopic rod, the spring is disposed between the limiting portion and the installing groove; when the photoelectric sensor is not triggered, the electric resection loop assembly operates normally; and when the photoelectric sensor is triggered, the electric resection loop assembly does not operate.

Preferably, an operating button is disposed on the operating handle, and a signal adapter board with an angle sensor is disposed in the operating handle.

Preferably, the operating handle is arranged behind the operating unit, a plug assembly is connected to a rear side of the operating handle through a guide tube, and the plug assembly includes a power plug and a light source plug.

The beneficial effects of the present disclosure are described as follows. The present disclosure utilizes an electronic endoscope combined with a plasma electric resection loop. An electronic imaging system is mature, cost-effective, and simple in process. The electronic endoscope offers a large field of view angle, providing a wide visual range. Furthermore, the image sensor is small in size, allowing for a reduction in an overall outer diameter, and further minimizing patient discomfort. Alternatively, under the same outer diameter, the inflow and outflow area can be increased, enhancing the clarity of the field of view. The image sensor provides accurate image orientation and strong directional perception, making the operation simple and intuitive. The protective glass with the cut-off coating is provided to eliminate or reduce the impact of light pollution generated by the plasma electric resection loop on the image sensor during operation. The distance sensor is provided to ensure that the electric resection loop is activated only at a specific distance from the tip end of the electronic endoscope and solve the problem of thermal damage to the tip end due to high temperature of the electric resection loop during misoperation when the electric resection loop is in excessive proximity to the tip end of the electronic endoscope. The angle sensor is provided to correct an image during a rotation operation, thereby achieving an image orientation functionality. Further, the lens tube with a matching structure is fabricated by utilizing a characteristic of the image sensor having a large tip end and a thin wire at a rear end, allowing for an increase of the water inflow area. In this way, in one aspect, thin blood caused by the surgery can be promptly replaced, maintaining a clear field of view. In another aspect, a resected tissue can be promptly discharged, preventing the blockage of the channel.

Features and advantages of the present disclosure will be described in detail by means of embodiments in conjunction with accompanying drawings.

### BRIEF DESCRIPTION OF THE DRAWINGS

FIG. 1 is a schematic structural diagram of the present disclosure;
FIG. 2 is a schematic diagram of an electric resection loop assembly of the present disclosure;
FIG. 3 is a schematic diagram of an operating unit of the present disclosure;
FIG. 4 is a schematic diagram illustrating installation of an image sensor of the present disclosure;
FIG. 5 is a schematic diagram of a distal tip of the present disclosure;
FIG. 6 is a schematic structural diagram of a lens barrel of the present disclosure;
FIG. 7 is a schematic diagram of an inflow/outlet channel of the present disclosure;
FIG. 8 is a schematic diagram of a distance sensor of the present disclosure; and
FIG. 9 is a schematic structural diagram of an operating handle of the present disclosure.

In the figures: 1, sheath assembly; 2, operating unit; 3, electric resection loop assembly; 4, electronic endoscope; 5, distance sensor; 6, distal tip; 7, operating handle; 8, power plug; 9, light source plug; 11, outer sheath; 12, inner sheath; 13, outflow channel; 14, inflow channel; 31, cutting tip; 32, connecting rod; 33, electrode rod; 34, slider; 35, finger hook; 41, lens tube; 42, conduit; 43, lens barrel; 44, image sensor; 45, protective glass; 46, inclined surface; 51, photoelectric sensor; 52, spring; 53, fixed rod; 54, telescopic rod; 55, installing groove; 56, limiting portion; 61, installing slot; 62, limiting groove; 63, first bonding surface; 64, second bonding surface; 65, light guide bundle; and 71, signal adapter board.

### DETAILED DESCRIPTION

### Embodiment 1

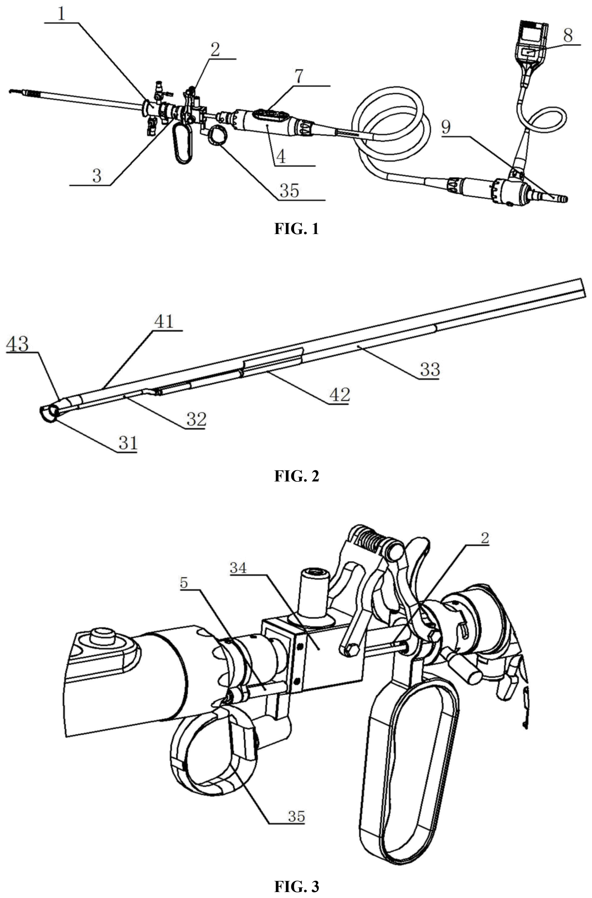

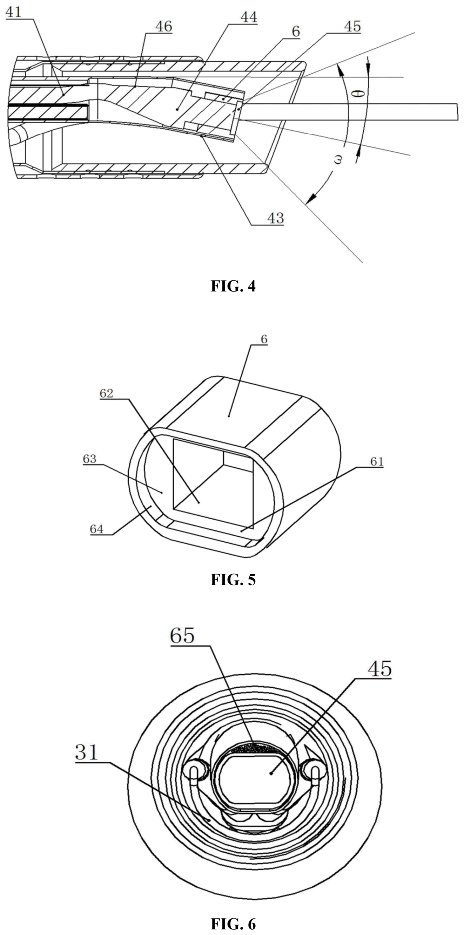

Referring to FIG. 1 to FIG. 7, a plasma resectoscope includes a sheath assembly 1, an operating unit 2, an electric resection loop assembly 3 and an electronic endoscope 4, wherein the sheath assembly 1 is disposed at a front end of the operating unit 2, the sheath assembly 1 includes an outer sheath 11 and an inner sheath 12, the outer sheath 11 is sleeved on an outer side of the inner sheath 12, the electronic endoscope 4 includes an lens tube 41 and an operating handle 7, the lens tube 41 is disposed within the inner sheath 12, the electric resection loop assembly 3 includes a cutting tip 31, a connecting rod 32 and an electrode rod 33, the cutting tip 31 is disposed on a front side of the lens tube 41, two sides of the cutting tip 31 are connected to the connecting rod 32, the connecting rod 32 is connected to the electrode rod 33, the electrode rod 33 is disposed on two sides of the lens tube 41, a slider 34 is connected to a tail end of a rear side of the electrode rod 33, the slider 34 is slidably installed on the operating unit 2, a finger hook 35 is connected to the slider 34, and a distance sensor 5 sensing a position of the slider 34 is disposed on the operating unit 2.

A conduit 42 is disposed on two sides of the lens tube 41, the electrode rod 33 is slidably sleeved in the conduit 42, a lens barrel 43 is disposed at a distal end of the lens tube 41, an image sensor 44 is disposed in the lens barrel 43, a protective glass 45 is disposed on an outer surface of the lens barrel 43, and a cut-off film is disposed on one side of the protective glass 45 close to the image sensor 44.

A distal tip 6 is disposed within the lens barrel 43, the image sensor 44 is installed in the distal tip 6, a diameter of the distal end of the image sensor 44 is greater than a diameter of the proximal end of the image sensor 44, a diameter of the lens barrel 43 is greater than a diameter of the lens tube 41, and the lens barrel 43 and the lens tube 41 are in smooth transition by means of an inclined surface 46.

An installing slot 61 for the protective glass 45 is provided in a surface of the distal tip 6, a limiting groove 62 for installing a lens barrel 43 is provided in the middle of the installing slot 61, a first bonding surface 63 and a second bonding surface 64 are disposed in the installing slot 61, a left side and a right side of the installing slot 61 are of an arc-shaped structure, the first bonding surface 63 is bonded to a periphery of the bottom surface of the protective glass 45, the second bonding surface 64 is bonded to a side surface of the protective glass 45, and a light guide bundle 65 for illumination is disposed above the distal tip 6.

The lens barrel 43 is bent downwards and forms a viewing angle θ with a central axis of the inner sheath 12, and an installing direction of the image sensor 44 is consistent with a bending direction of the lens barrel 43.

The viewing angle θ of the image sensor 44 ranges from 0 to 30° to adjust an observation range.

A field of view angle ω of the image sensor 44 is selectable between 90° and 120°.

A gap between the inner sheath 12 and outer sheath 11 is an outflow channel 13, and a gap between the inner sheath 12 and the lens tube 41 is an inflow channel 14.

The operating handle 7 is arranged behind the operating unit 2, a plug assembly is connected to a rear side of the operating handle 7 through a guide tube, and the plug assembly includes a power plug 8 and a light source plug 9.

### Embodiment 2

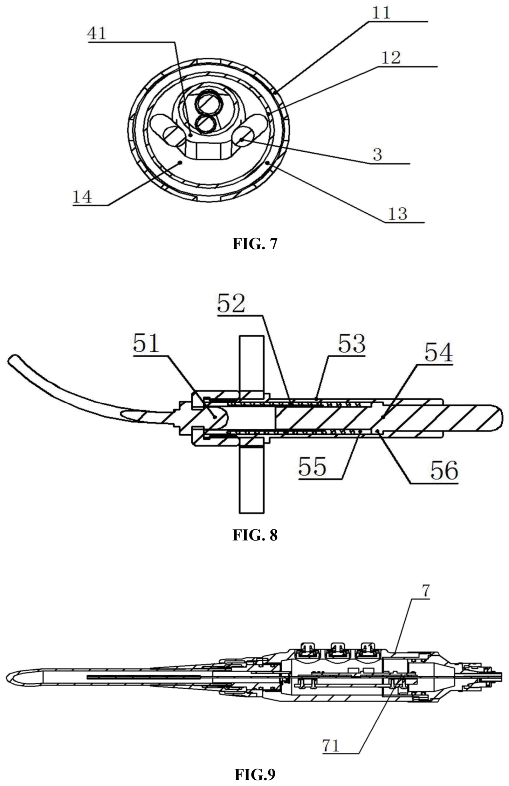

Referring to FIG. 8, a plasma resectoscope is provided, wherein the distance sensor 5 includes a photoelectric sensor 51, a spring 52, a fixed rod 53 and a telescopic rod 54, wherein the fixed rod 53 is fixed on the operating unit 2, an installing groove 55 is disposed within the fixed rod 53, the photoelectric sensor 51 is disposed on one end of the installing groove 55 away from the slider 34, the telescopic rod 54 is slidably installed on one end of the installing groove 55 close to the slider 34, a tail end of the telescopic rod 54 protrudes beyond the fixed rod 53, a limiting portion 56 is disposed on the telescopic rod 54, a spring 52 is disposed between the limiting portion 56 and the installing groove 55, when the photoelectric sensor 51 is not triggered, the electric resection loop assembly 3 operates normally, and when the photoelectric sensor 51 is triggered, the electric resection loop assembly 3 does not operate.

When the operating unit 2 does not operate or has not reached a designated position, the telescopic rod 54 is squeezed by the slider 34, the spring 52 is compressed, and a tail portion of the telescopic rod 54 is sensed by the photoelectric sensor 51. The electric resection loop assembly 3 does not operate even if an operating switch for controlling the electric resection loop assembly 3 is turned on. The plasma electric resection loop can operate normally only when the photoelectric sensor 51 is not triggered.

All other aspects are the same as those described in the above-mentioned embodiment 1.

### Embodiment 3

Referring to FIG. 9, a plasma resectoscope is provided, wherein an operating button is disposed on the operating handle 7, and a signal adapter board 71 with an angle sensor is disposed in the operating handle 7.

All other aspects are the same as those described in the above-mentioned embodiment 1.

The operating button includes a photo button, a video recording button, and a vascular recognition enhancement button, enabling functions such as taking photos, recording videos or enhancing vascular recognition.

During use, a rotation angle of the electronic endoscope 4 is sensed by the angle sensor, and the captured image is processed by adopting a corresponding image rotation algorithm according to the angle data provided by the angle sensor. The image is rotated by calculating a new position of each pixel in the image after rotation according to a common algorithm such as a rotation algorithm based on matrix transformation, thereby correcting an angular deviation of the image due to the rotation of the camera, and further maintaining a picture in a predetermined direction.

A virtual interface synchronized with a real interface exists in an image correction system. A cross-shaped interface or a flat-shaped interface is disposed on the virtual interface. Such a specific interface (that is, the cross-shaped interface or the flat-shaped interface) is a key element configured to detect and correct an image deviation. Moreover, a stationary cross-shaped reference interface used as a reference standard exists in the image correction system as well. The image correction system senses an angular difference between the cross-shaped interface or the flat-shaped interface on the virtual interface and the cross-shaped reference interface via an angle sensor. When an image is offset, the virtual interface changes accordingly, resulting in an angular deviation between the cross-shaped interface or the flat-shaped interface on the virtual interface and the cross-shaped reference interface. The angle sensor can accurately measure such an angular deviation, and convert the angular deviation into processable information such as an electric signal or digital signal. The image correction system may calculate a direction and an extent of image offset according to angular information fed back by the sensor, and then correct the image by a specific algorithm and a control mechanism, such that images in the real interface and the virtual interface can be restored to their correct positions and angles, thereby achieving the purpose of the image correction.

The image correction system stores a relational expression characterizing a correspondence between an angle generated by the cross-shaped interface or the flat-shaped interface on the virtual interface and the stationary interface and an angle of the angle sensor across a 360° range, and when an actual angle of the angle sensor is input into the image correction system, the image correction system outputs a corresponding correction value according to the relational expression to correct the angle.

Particularly, the angle sensor (such as a MPU6050 gyroscope) is connected to an Arduino reading data from the angle sensor and calculating a rotation angle; angle data read by the Arduino is then transmitted to a computer; and Python and OpenCV libraries are used to control a camera to acquire an image, and then the image is rotated according to the received angle data to ensure the image is displayed with the correct orientation on a display screen of the computer

The present disclosure adopts an electronic endoscope for plasma resection, filling a gap in this field.

The above-mentioned embodiments are provided to illustrate the present disclosure, and are not intended to limit the present disclosure. Any simple transformations of the present disclosure fall within the protection scope of the present disclosure.

## Claims

1. A plasma resectoscope, comprising a sheath assembly (1), an operating unit (2), an electric resection loop assembly (3) and an electronic endoscope (4), wherein the sheath assembly (1) is disposed at a front end of the operating unit (2), the sheath assembly (1) comprises an outer sheath (11) and an inner sheath (12), the outer sheath (11) is sleeved on an outer side of the inner sheath (12); the electronic endoscope (4) comprises a lens tube (41) and an operating handle (7), the lens tube (41) is disposed within the inner sheath (12), the electric resection loop assembly (3) comprises a cutting tip (31), a connecting rod (32) and an electrode rod (33); the cutting tip (31) is disposed on a distal end of the lens tube (41); two sides of the cutting tip (31) are connected to the connecting rod (32), the connecting rod (32) is connected to the electrode rod (33), and the electrode rod (33) is disposed on two sides of the lens tube (41); a slider (34) is connected to a tail end of a rear side of the electrode rod (33), the slider (34) is slidably installed on the operating unit (2), a finger hook (35) is connected to the slider (34), and a distance sensor (5) sensing a position of the slider (34) is disposed on the operating unit (2); a conduit (42) is disposed on two sides of the lens tube (41), the electrode rod (33) is slidably sleeved in the conduit (42), a lens barrel (43) is provided at the distal end of the lens tube (41), and an image sensor (44) is disposed in the lens barrel (43); a distal tip (6) is disposed within the lens barrel (43), the image sensor (44) is installed in the distal tip (6), a diameter of the distal end of the image sensor (44) is greater than a diameter of a proximal end of the image sensor (44), a diameter of the lens barrel (43) is greater than a diameter of the lens tube (41), and the lens barrel (43) and the lens tube (41) are in smooth transition by means of an inclined surface (46); a protective glass (45) is disposed on an outer surface of the lens barrel (43), and a cut-off film is disposed on one side of the protective glass (45) close to the image sensor (44); an installing slot (61) for the protective glass (45) is provided in a surface of the distal tip (6), a limiting groove (62) for installing the lens barrel (43) is provided in the middle of the installing slot (61), a first bonding surface (63) and a second bonding surface (64) are disposed in the installing slot (61), a left side and a right side of the installing slot (61) are of an arc-shaped structure, the first bonding surface (63) is bonded to the bottom surface of the protective glass (45), and the second bonding surface (64) is bonded to a side surface of the protective glass (45); an operating button is disposed on the operating handle (7), a signal adapter board (71) with an angle sensor is disposed in the operating handle (7), and a virtual interface synchronized with a real interface exists in an image correction system; a cross-shaped interface or a flat-shaped interface is disposed on the virtual interface and configured to detect and correct an image deviation; moreover, a stationary cross-shaped reference interface used as a reference standard is disposed on the virtual interface; the image correction system senses an angular difference between the cross-shaped interface or the flat-shaped interface on the virtual interface and the stationary cross-shaped reference interface via the angle sensor; when an image is offset, the virtual interface changes accordingly, resulting in an angular deviation between the cross-shaped interface or the flat-shaped interface on the virtual interface and the cross-shaped reference interface; and the image correction system stores a relational expression characterizing a correspondence between an angle generated by the cross-shaped interface or the flat-shaped interface on the virtual interface and the stationary interface and an angle of the angle sensor across a 360° range, and when an actual angle of the angle sensor is input into the image correction system, the image correction system outputs a corresponding correction value according to the rational expression to correct the angle.

2. The plasma resectoscope of claim 1, wherein a light guide bundle (65) for illumination is disposed above the distal tip (6).

3. The plasma resectoscope of claim 1, wherein the lens barrel (43) is bent downwards and forms a viewing angle θ with a central axis of the inner sheath (12), and an installing direction of the image sensor (44) is consistent with a bending direction of the lens barrel (43).

4. The plasma resectoscope of claim 3, wherein the viewing angle θ ranges from 0 to 30°.

5. The plasma resectoscope of claim 1, wherein a gap between the inner sheath (12) and the outer sheath (11) is an outflow channel (13), and a gap between the inner sheath (12) and the lens tube (41) is an inflow channel (14).

6. The plasma resectoscope of claim 1, wherein the distance sensor (5) comprises a photoelectric sensor (51), a spring (52), a fixed rod (53) and a telescopic rod (54), wherein the fixed rod (53) is fixed on the operating unit (2), an installing groove (55) is disposed within the fixed rod (53), the photoelectric sensor (51) is disposed on one end of the installing groove (55) away from the slider (34), the telescopic rod (54) is slidably installed on one end of the installing groove (55) close to the slider (34), a tail end of the telescopic rod (54) protrudes beyond the fixed rod (53), a limiting portion (56) is disposed on the telescopic rod (54), the spring (52) is disposed between the limiting portion (56) and the installing groove (55); when the photoelectric sensor (51) is not triggered, the electric resection loop assembly (3) operates normally; and when the photoelectric sensor (51) is triggered, the electric resection loop assembly (3) does not operate.

7. The plasma resectoscope of claim 1, wherein the operating handle (7) is arranged behind the operating unit (2), a plug assembly is connected to a rear side of the operating handle (7) through a guide tube, and the plug assembly comprises a power plug (8) and a light source plug (9).

8. The plasma resectoscope of claim 7, wherein the angle sensor is connected to an Arduino reading data from the angle sensor and calculating a rotation angle; angle data read by the Arduino is then transmitted to a computer; and Python and OpenCV libraries are used to control a camera to acquire an image, and then the image is rotated according to the received angle data to achieve directional display of the image on a display screen of the computer.
